(19) Europäisches Patentamt
European Patent Office
Office européen des brevets

(11) Veröffentlichungsnummer: **0 007 479**
**B1**

(12) # EUROPÄISCHE PATENTSCHRIFT

(45) Veröffentlichungstag der Patentschrift: **29.12.82**

(21) Anmeldenummer: **79102257.7**

(22) Anmeldetag: **04.07.79**

(51) Int. Cl.³: **C 07 D 207/04**, C 07 D 211/06, C 07 D 217/04, C 07 D 223/00, C 07 D 265/28, C 07 D 295/04, A 01 N 43/00

(54) **N-Arylpropyl-substituierte cyclische Amine, Fungizide, die diese Verbindungen enthalten und Verfahren zu ihrer Herstellung.**

(30) Priorität: **08.07.78 DE 2830127**

(43) Veröffentlichungstag der Anmeldung: **06.02.80 Patentblatt 80/3**

(45) Bekanntmachung des Hinweises auf die Patenterteilung: **29.12.82 Patentblatt 82/52**

(84) Benannte Vertragsstaaten: **AT BE CH DE FR GB IT LU NL SE**

(56) Entgegenhaltungen:
**EP-A-0 000 333**
**DE-A-2 656 747**
**DE-A-2 752 096**
**DE-A-2 752 135**
**GB-A-984 119**
**LU-A-79 385**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Buschmann, Ernst, Dr.-Chem., An der Froschlache 7, D-6700 Ludwigshafen (DE)**
Erfinder: **Zeeh, Bernd, Dr.-Chem., Thorwaldsenstrasse 5, D-6700 Ludwigshafen (DE)**
Erfinder: **Pommer, Ernst-Heinrich, Dr., Berliner Platz 7, D-6703 Limburgerhof (DE)**
Erfinder: **Goetz, Norbert, Dr.-Chem., Schoefferstrasse 25, D-6520 Worms (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 007 479 B1**

BUNDESDRUCKEREI BERLIN

N-Arylpropyl-substituierte cyclische Amine, Fungizide, die diese Verbindungen enthalten, und Verfahren zu ihrer Herstellung

Die vorliegende Erfindung betrifft neue wertvolle N-Arylpropyl-substituierte Amine sowie ihre Salze, Molekül- und Additionsverbindungen mit guter fungizider Wirkung, Fungizide, die diese Verbindungen enthalten, und Verfahren zur Herstellung dieser Verbindungen.

Es ist bekannt, N-Tridecyl-2,6-dimethyl-morpholin, seine Salze und seine Molekül- und Additionsverbindungen als Fungizide zu verwenden (DE-PS 1 164 152, DE-PS 1 173 722, DE-OS 2 461 513). Seine fungizide Wirkung und Verträglichkeit gegenüber Kulturpflanzen wird jedoch nicht allen Anforderungen der Praxis gerecht.

Es wurde gefunden, daß N-Arylpropyl-substituierte cyclische Amine der Formel

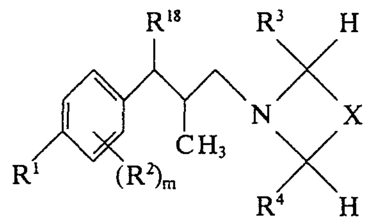

in der
$R^1$ einen tertiären Alkylrest,
$R^2$ Chlor, Brom oder Fluor,
$m = 1$ oder 2,
$R^3$ und $R^4$ je ein Wasserstoffatom,
X die Reste

$$-CH_2-CH_2-CH_2 \quad \text{und} \quad -\underset{R^{14}}{\underset{|}{CH}}-O-\underset{R^{15}}{\underset{|}{CH}}- \quad \text{und}$$

$R^{14}$ und $R^{15}$ je einen niederen Alkylrest bedeuten,
$R^{18}$ ein Wasserstoffatom bedeutet,
sowie ihre Salze

eine gute fungizide Wirkung haben, die der Wirkung der bekannten Morpholinderivate überlegen ist.

Salze sind beispielsweise die Salze mit anorganischen Säuren, z. B. Chloride, Fluoride, Bromide, Jodide, Sulfate, Nitrate, Phosphate, Acetate, Propionate, Molekül- oder Additionsverbindungen entstehen beispielsweise mit Säuren von Tensiden, z. B. Dodecylbenzolsulfonsäure.

Die neuen Verbindungen können, soweit es sich um die 2,6-Dimethylmorpholine handelt, als cis- und trans-Isomere isoliert werden.

Das Verfahren zur Herstellung der neuen Verbindungen ist dadurch gekennzeichnet, daß man ein Halogenid der Formel

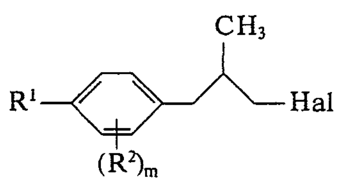

(II)

worin $R^1$, $R^2$ und m die oben angegebenen Bedeutungen haben und Hal, Chlor oder Brom bedeutet, umsetzt mit einer Verbindung der Formel

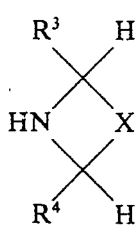

(III)

worin $R^3$, $R^4$ und X die oben angegebenen Bedeutungen haben.

Die Umsetzung wird beispielsweise in höhersiedenden Lösungsmitteln oder ohne Lösungsmittel im Temperaturbereich von 80 bis 200° C durchgeführt.

Bevorzugt wird die Umsetzung ohne Lösungsmittel bei einer Temperatur von 100 bis 160° C.

Die Halogenide der entsprechenden Formel II werden erhalten durch Alkylierung von Phenylpropylhalogeniden der Formel

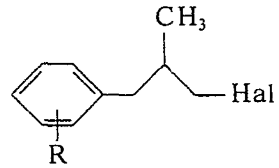

(IV)

in der R Wasserstoff oder ein Fluoratom und Hal Brom oder Chlor bedeuten, mit einem Olefin, Alkohol oder Alkylhalogenid in Gegenwart eines sauren Katalysators und gegebenenfalls durch anschließende Umsetzung mit Chlor oder Brom.

Die folgenden Beispiele erläutern die Herstellung der neuen Verbindungen.

## Beispiel 1

Eine Lösung von 23 g 2,6-cis-Dimethylmorpholin und 30,4 g 3-(p-tert.-Butyl-o-chlor-phenyl)-2-methyl-propylbromid in 150 ml Acetonitril wird 4 Stunden unter Rückfluß erhitzt. Das Rohprodukt wird in Methylenchlorid gelöst, die Lösung mit Wasser, wäßriger Natriumbicarbonatlösung und wieder mit Wasser gewaschen, über $Na_2SO_4$ getrocknet, das Lösungsmittel verdampft und der Rückstand destilliert. Man erhält 22 g N-[3-(p-tert.-Butyl-o-chlor-phenyl)-2-methyl-propyl]-2,6-cis-dimethyl-morpholin (Wirkstoff Nr. 1), $Kp_{0,05} = 133$ bis 135° C.

In entsprechender Weise erhält man die folgenden Wirkstoffe:

| Wirkstoff Nr. | |
|---|---|
| 2 | N-[3-(p-tert.-Butyl-o-brom-phenyl)-2-methyl-propyl]-2,6-cis-dimethyl-morpholin, $Kp_{0,05} = 145$ bis 147° C |
| 3 | N-[3-(p-tert.-Amyl-o-brom-phenyl)-2-methyl-propyl]-2,6-cis-dimethyl-morpholin, $Kp_{0,2} = 183$ bis 187° C |
| 4 | N-[3-(p-tert.-Butyl-o-chlor-phenyl)-2-methyl-propyl]-2,6-cis-dimethyl-morpholin-hydrochlorid, Schmp. 240 bis 242° C (Zersetzung) |
| 5 | N-[3-(p-tert.-Butyl-o-brom-phenyl)-2-methyl-propyl]-2,6-cis-dimethyl-morpholin-hydrochlorid, Schmp. 244 bis 246° C (Zersetzung) |
| 6 | Gemisch aus N-(3-p-tert.-Amyl-o-chlor-phenyl-2-methyl-propyl)-2,6-cis-dimethylmorpholin und N-(3-p-tert.-Amyl-m-chlor-phenyl-2-methyl-propyl)-2,6-cis-dimethyl-morpholin, $Kp_{0,1} = 152$ bis 160° C |
| 7 | N-[3-(p-tert.-Butyl-o-chlor-phenyl)-2-methyl-propyl]-piperidin, $Kp_{0,1} = 122$ bis 128° C |
| 8 | N-[3-(p-tert.-Butyl-o-chlor-phenyl)-2-methyl-propyl]-3,5-diethyl-morpholin. |

Die erfindungsgemäßen Wirkstoffe und die entsprechenden Fungizide sind insbesondere geeignet zur Bekämpfung von Pflanzenkrankheiten, z. B. Erysiphe graminis (echter Mehltau) an Getreide, Erysiphe chichoriacèarum (echter Mehltau) an Kürbisgewächsen, Podosphaera leucotricha an Äpfeln, Unicinula necator an Rosen, Microsphaera querci an Eichen, Botrytis cinerea an Erdbeeren, Reben, Mycosphaerella musicola an Bananen, Puccinia-Arten (Rostpilze) an Getreide, Uromyces appendiculatus und U. phaseoli an Bohnen, Hemileia vastatrix an Kaffee und Rhizoctonia solani. Sie sind systemisch wirksam; sie werden sowohl über die Wurzeln als auch über die Blätter aufgenommen und im Pflanzengewebe transportiert.

Bei der Anwendung der neuen Wirkstoffe zur Behandlung von Pflanzen gegen Pilzinfektionen liegen die Aufwandmengen zwischen 0,01 und 4 kg Wirkstoff/ha Fläche. Zum Oberflächenschutz von Bäumen oder Früchten können die Wirkstoffe auch in Verbindung mit Kunststoffdispersionen 0,25%ig bis 5%ig, bezogen auf das Gewicht der Dispersion, verwendet werden.

Die Fungizide enthalten im allgemeinen zwischen 0,1 und 95 Gewichtsprozent Wirkstoff, vorzugsweise zwischen 0,5 und 90 Prozent. Die Anwendung erfolgt beispielsweise durch Gießen, Spritzen, Stäuben, Pudern, Streichen, Tauchen oder Sprühen.

Die Wirkstoffe können mit anderen bekannten Fungiziden gemischt werden. In vielen Fällen erhält man dabei eine Vergrößerung des fungiziden Wirkungsspektrums; bei einer Anzahl von Fungizidmischungen in den Gewichtsverhältnissen 1 : 10 bis 10 : 1 treten auch synergistische Effekte auf, d. h. die fungizide Wirksamkeit des Kombinationsproduktes ist größer als die der addierten Wirksamkeiten der Einzelkomponenten. Funigzide, die mit den erfindungsgemäßen Verbindungen kombiniert werden können, sind beispielsweise:

Dithiocarbamate und deren Derivate, wie
Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramidsulfid,
Ammoniak-Komplex von Zink-(N,N-äthylen-bis-dithiocarbamat)
und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat)
und
N,N'-Polypropylen-bis-(thiocarbamoyl)-disulfid;
heterocyclische Verbindungen, wie
N-Trichlormethylthio-tetrahydrophthalimid,
N-Trichlormethylthio-phthalimid,
N-(1,1,2,2-Tetrachloräthylthio)-tetrahydrophthalimid,
1-(Butylcarbamoyl)-2-benzimidazol-carbaminsäuremethylester,
2-Methyloxycarbonylamino-benzimidazol,
2,3-Dihydro-5-carboxanilido-6-methyl-1,4-oxathiin-4,4-dioxid,
2,3-Dihydro-5-carboxaanilido-6-methyl-1,4-oxathiin,
5-Butyl-2-dimethylamino-4-hydroxy-6-methyl-pyrimidin,
1,2-Bis-(3-äthoxycarbonyl-2-thioureido)-benzol,
1,2-Bis-(3-methoxycarbonyl-2-thioureido)-benzol,
und verschiedene andere Fungizide wie
Dodecylguanidinacetat,
N-Dichlorfluormethyl-thio-N',N'-dimethyl-N-phenylschwefelsäurediamid,
2,5-Dimethyl-furan-3-carbonsäureanilid,
2,5-Dimethyl-furan-3-carbonsäure-cyclohexylamid,
2,5-Dimethyl-furan-3-carbonsäure-N-methoxy-cyclohexylamid,
2-Jodbenzoesäureanilid,
2-Brombenzoesäureanilid,
3-Nitro-isophthalsäure-diisopropylester,
1-(1,2,4-Triazolyl-1')-[1-(4'-chlorphenoxy)]-3,3-dimethylbutan-2-on,
1-(1,2,4-Triazolyl-1')-[1-(4'-chlorphenoxy)]-3,3-dimethylbutan-2-ol,
1-(1-Imidazolyl)-2-allyloxy-2-(2,4-dichlorphenyl)-äthan,
Piperazin-1,4-diyl-bis-1-(2,2,2-trichloräthyl)-formamid,
2,4,5,6-Tetrachlor-isophthalonitril,
1,2-Dimethyl-3,5-diphenyl-pyrazoliniummethylsulfat.

Die Anwendung erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoron usw., stark plare Lösungsmittel, z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:
Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholgylkoläther,

Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol-, Octylphenol-, Nonylphenol, Alkylphenolpolyglykoläther. Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Kreide, Bolus, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte wie Getreidemehle, Baumrinden-, Holz- und Nußschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z. B. Silikone), Wachstumsregulatoren, Antidotmittel oder andere wirksame Verbindungen, zugesetzt werden.

Für die folgenden Versuche wurden folgende bekannte Vergleichssubstanzen verwendet.

Wirkstoff Nr.

A

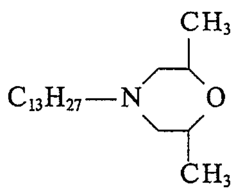

(bekannt)

B

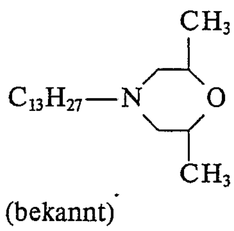 · $CH_3COOH$

(bekannt)

C

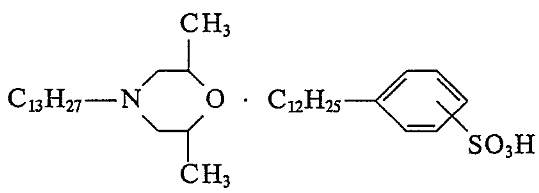

(bekannt)

## Beispiel 2

Blätter von in Töpfen gewachsenen Weizenkeimlingen der Sorte »Jubilar« werden mit wäßrigen Emulsionen aus 80% (Gewichtsprozent) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

| Wirkstoff | Befall der Blätter nach Spritzungen mit x %iger Wirkstoffbrühe | | | |
|---|---|---|---|---|
| | x = 0,006 | 0,012 | 0,025 | 0,05 |
| 1 | 0 | 0 | 0 | 0 |
| 4 | 0 | 0 | 0 | 0 |
| A (bekannt) | 3–4 | 3 | 2 | 1 |
| B (bekannt) | 4 | 4 | 2 | 1 |
| C (bekannt) | 2 | 1 | 1 | 0 |
| Kontrolle (unbehandelt) | 4 | | | |

0 = kein Befall, abgestuft bis 5 = Totalbefall.

Beispiel 3

In einem weiteren Versuch werden, wie in Beispiel 3 beschrieben, Blätter von in Töpfen gewachsenen Gerstenkeimlingen der Sorte »Firlbecks Union« behandelt und mit Sporen (Konidien) des Gerstenmehltaus (Erysiphe graminis var. hordei) bestäubt.

| Wirkstoff | Befall der Blätter nach Spritzungen mit x %iger Wirkstoffbrühe | | |
|---|---|---|---|
| | x = 0,006 | 0,012 | 0,025 |
| 1 | 0 | 0 | 0 |
| 4 | 0 | 0 | 0 |
| A (bekannt) | 2 | 1 | 0–1 |
| B (bekannt) | 3 | 1 | 1 |
| C (bekannt) | 1 | 0 | 0 |
| Kontrolle (unbehandelt) | 4 | | |

0 = kein Befall, abgestuft bis 5 = Totalbefall.

Beispiel 4

Blätter von in Töpfen gewachsenen Weizenpflanzen werden mit Sporen des Weizenbraunrostes (Puccinia recondita) künstlich infiziert und 48 Stunden lang bei 20 bis 25°C in einer wasserdampfgesättigten Kammer aufgestellt. Danach werden die Pflanzen mit wäßrigen Spritzbrühen, die in dem Wasser gelöst oder emulgiert eine Mischung aus 80% des zu prüfenden Wirkstoffes und 20% Natriumligninsulfonat enthalten, besprüht und im Gewächshaus bei Temperaturen zwischen 20 und 22°C und bei 75 bis 80% relativer Luftfeuchtigkeit aufgestellt. Nach 10 Tagen wird das Ausmaß der Rostpilzentwicklung beurteilt.

| Wirkstoff | Befall der Blätter nach Spritzungen mit x %iger Wirkstoffbrühe | | | |
|---|---|---|---|---|
| | x = 0,012 | 0,025 | 0,05 | 0,1 |
| 1 | 0 | 0 | 0 | 0 |
| 4 | 0 | 0 | 0 | 0 |
| A bekannt | 4 | 4 | 3 | 2 |
| B bekannt | 4 | 4 | 4 | 3 |
| C bekannt | 4 | 4 | 3 | 3 |
| Kontrolle (unbehandelt) | 4 | | | |

0 = kein Befall, abgestuft bis 5 = Totalbefall.

### Beispiel 5

Man vermischt 90 Gewichtsteile der Verbindung 4 mit 10 Gewichtsteilen N-Methyl-alpha-pyrrolidon und erhält eine Lösung, die zur Anwendung in Form kleinster Tropfen geeignet ist.

### Beispiel 6

20 Gewichtsteile der Verbindung 4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenyol und 10 Gewichtsteilen des Anlagerungsprdukts von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

### Beispiel 7

3 Gewichtsteile der Verbindung 4 werden mit 97 Gewichtsteilen feinteiligem Kaolin innig vermischt. Man erhält auf diese Weise ein Stäubemittel, das 3 Gewichtsprozent des Wirkstoffs enthält.

### Beispiel 8

40 Gewichtsteile des Wirkstoffs 4 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion, die 0,04 Gewichtsprozent Wirkstoff enthält.

**Patentansprüche für die Vertragsstaaten: BE, CH, DE, FR, GB, IT, LU, NL, SE**

1. N-Arylpropyl-substituiertes cyclisches Amin der Formel

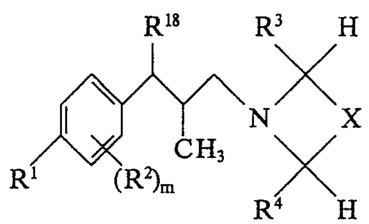

in der
$R^1$ einen tertiären Alkylrest,

$R^2$ Chlor, Brom oder Fluor,
m = 1 oder 2,
$R^3$ und $R^4$ je ein Wasserstoffatom,
X die Reste

$$-CH_2-CH_2-CH_2- \quad \text{und} \quad \underset{R^{14}}{-CH}-O-\underset{R^{15}}{CH-} \quad \text{und}$$

$R^{14}$ und $R^{15}$ je einen niederen Alkylrest bedeuten,
$R^{18}$ ein Wasserstoffatom bedeutet, sowie ihre Salze.

2. Fungizid, enthaltend ein N-Arylpropyl-substituiertes cyclisches Amin gemäß Anspruch 1.
3. Fungizid, enthaltend einen festen oder flüssigen Trägerstoff und ein N-Arylpropyl-substituiertes cyclisches Amin gemäß Anspruch 1.
4. Verfahren zur Herstellung eines Fungizids, dadurch gekennzeichnet, daß man einen festen oder flüssigen Trägerstoff vermischt mit einem N-Arylpropyl-substituierten cyclischen Amin gemäß Anspruch 1.

**Patentansprüche für den Vertragsstaat AT**

1. Fungizid, enthaltend ein N-Arylpropyl-substituiertes cyclisches Amin der Formel

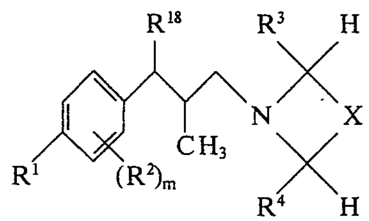

in der
$R^1$ einen tertiären Alkylrest,
$R^2$ Chlor, Brom oder Fluor,
m = 1 bis 2,
$R^3$ und $R^4$ je ein Wasserstoffatom,
X die Reste

$$-CH_2-CH_2-CH_2 \quad \text{und} \quad \underset{R^{14}}{-CH}-O-\underset{R^{15}}{CH-} \quad \text{und}$$

$R^{14}$ und $R^{15}$ je einen niederen Alkylrest bedeuten,
$R^{18}$ ein Wasserstoffatom bedeutet, sowie ihre Salze.

2. Fungizid, enthaltend einen festen oder flüssigen Trägerstoff und ein N-Arylpropyl-substituiertes cyclisches Amin wie in Anspruch 1 definiert.
3. Verfahren zur Herstellung eines Fungizids, dadurch gekennzeichnet, daß man einen festen oder flüssigen Trägerstoff vermischt mit einem N-Arylpropyl-substituierten cyclischen Amin wie in Anspruch 1 definiert.
4. Verfahren zur Bekämpfung von Pilzen, dadurch gekennzeichnet, daß man die Pflanzen behandelt mit einem N-Arylpropyl-substituierten cyclischen Amin wie in Anspruch 1 definiert.

**Claims for the contracting states: BE-CH-DE-FR-GB-IT-LU-NL-SE**

1. An N-arylpropyl-substituted cyclic amine of the formula

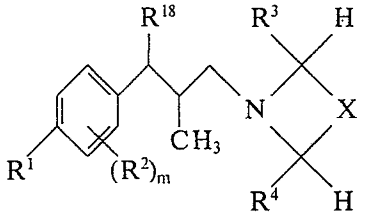

where $R^1$ is tertiary alkyl, $R^2$ is chlorine, bromine or fluorine, m denotes 1 or 2, $R^3$ and $R^4$ each denote hydrogen, X denotes

$-CH_2-CH_2-CH_2-$ or 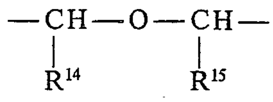

and $R^{14}$ and $R^{15}$ each denote lower alkyl, and $R^{18}$ is hydrogen, and its salts.

2. A fungicide containing an N-arylpropyl-substituted cyclic amine as claimed in claim 1.

3. A fungicide containing a solid or liquid carrier and an N-arylpropyl-substituted cyclic amine as claimed in claim 1.

4. A process for manufacturing a fungicide, wherein a solid or liquid carrier is mixed with an N-arylpropyl-substituted cyclic amine as claimed in claim 1.

**Claims for the contracting state AT**

1. A fungicide containing an N-arylpropyl-substituted cyclic amine of the formula

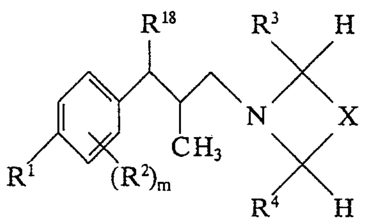

where $R^1$ is tertiary alkyl, $R^2$ is chlorine, bromine or fluorine, m denotes 1 or 2, $R^3$ and $R^4$ each denote hydrogen, X denotes

$-CH_2-CH_2-CH_2-$ or 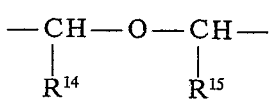

and $R^{14}$ and $R^{15}$ each denote lower alkyl, and $R^{18}$ is hydrogen, and its salts.

2. A fungicide containing a solid or liquid carrier and an N-arylpropyl-substituted cyclic amine as defined in claim 1.

3. A process for manufacturing a fungicide, wherein a solid or liquid carrier is mixed with an N-arylpropyl-substituted cyclic amine as defined in claim 1.

4. A process for combating fungi, wherein the plants are treated with an N-arylpropyl-substituted cyclic amine as defined in claim 1.

**Revendications pour les états contractants: BE-CH-DE-FR-GB-IT-LU-NL-SE**

1. Amine cyclique substitué par N-arylpropyle de formule

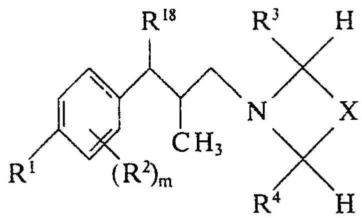

dans laquelle
$R^1$ représente un reste alkyle tertiaire,
$R^2$ chlore, brome ou fluor,
m = 1 ou 2,
$R^3$ et $R^4$ représentent chacun un atome d'hydrogène,
X les restes

$-CH_2-CH_2-CH_2-$ et 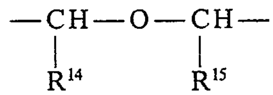 et

$R^{14}$ et $R^{15}$ représentent chacun un reste alkyle inférieur,
$R^{18}$ représente un atome d'hydrogène
et ses sels.

2. Fongicide contenant une amine cyclique substitué par N-arylpropyle selon la revendication 1.

3. Fongicide contenant un support solide ou liquide et une amine cyclique substitué par N-arylpropyle selon la revendication 1.

4. Procédé de préparation d'un fongicide, caractérisé par le fait qu'on mélange un support solide ou liquide avec une amine cyclique substitué par N-arylpropyle selon la revendication 1.

**Revendications pour l'état contractant AT**

1. Fongicide contenant une amine cyclique substitué par N-arylpropyle de formule

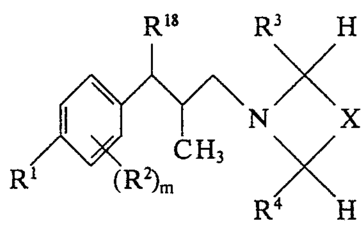

dans laquelle
$R^1$ représente un reste alkyle tertiaire,
$R^2$ chlore, brome ou fluor,
m = 1 ou 2,
$R^3$ et $R^4$ représentent chacun un atome d'hydrogène,
X les restes

$-CH_2-CH_2-CH_2-$ et 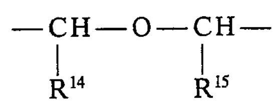 et

$R^{14}$ et $R^{15}$ représentent chacun un reste alkyle inférieur,
$R^{18}$ représente un atome d'hydrogène
et ses sels.

2. Fongicide contenant un support solide ou liquide et une amine cyclique substitué par N-arylpropyle selon la revendication 1.

3. Procédé de préparation d'un fongicide, caractérisé par le fait qu'on mélange un support solide ou liquide avec une amine cyclique substitué par N-arylpropyle selon la revendication 1.

4. Procédé de lutte contre les champignons, caractérisé par le fait qu'on traite les plantes avec une amine cyclique substitué par N-arylpropyle telle que définie dans la revendication 1.